# DEMANDE DE BREVET EUROPEEN

(11) **EP 3 392 653 A1**
(43) Date de publication de la demande: **24.10.2018**
(21) Numéro de dépôt: 18167320.3
(22) Date de dépôt: 13.04.2018
(51) Int. Cl.: G01N 33/24, G01N 1/22, G01N 33/00

(54) **DISPOSITIF ET PROCEDE DE MESURE DE CONCENTRATIONS DE GAZ ET DISPOSITIF ET PROCEDE DE PRELEVEMENTS D'ECHANTILLONS POUR MESURER DES FLUX DE GAZ EMIS PAR UNE SURFACE**

(30) Priorité: 20.04.2017 FR 1753455
(71) Demandeur: Institut National De L'environnement Industriel Et Des Risques, 60550 Verneuil-en-Halatte (FR)
(72) Inventeur: POKRYSZKA, Zbigniew, 60290 LAIGNEVILLE (FR)
(74) Mandataire: Cabinet Beau de Loménie

(57) **Abrégé**

Procédé de prélèvement d'échantillons pour mesurer des concentrations de gaz émis par une surface (15), ou de mesure de telles concentrations, suivant lequel :
on dispose une chambre (12) de telle sorte qu'elle délimite un volume fermé recouvrant ladite surface, et
on procède en alternance à :
a) des étapes de renouvellement, durant lesquelles sans déplacer la chambre on injecte de l'air extérieur dans celle-ci et on extrait des gaz contenus dans celle-ci afin de renouveler un mélange gazeux contenu dans la chambre ; et
b)
- soit des étapes de prélèvement d'échantillons dans lesquelles on prélève des échantillons des gaz contenus dans la chambre,
- soit des étapes de mesure, durant lesquelles on fait circuler le mélange gazeux contenu dans la chambre à travers un système d'analyse (14) qui mesure au moins deux fois la concentration dudit au moins un gaz.

Dispositif pour la mise en oeuvre du procédé.

## Description

### DOMAINE DE L'INVENTION

L'invention concerne un dispositif pour la mesure des émissions gazeuses d'une surface solide ou liquide (sol, roche, mur, dalle, eau, etc.) et un procédé pour la mesure de telles émissions.

### ARRIERE-PLAN DE L'INVENTION

Certaines surfaces, naturelles ou anthropiques, laissent échapper des gaz, notamment des gaz nocifs. C'est le cas par exemple de marécages, de terrains affectés par une activité volcanique, de terrains situés à l'aplomb de certaines mines souterraines, de décharges, ou encore de sols pollués. De même, la plupart des sols émettent naturellement du dioxyde de carbone produit par une activité biologique qui a lieu dans des couches superficielles de ces sols. Dans de nombreux cas, la quantité de gaz dégagés par ces surfaces doit être mesurée. On comprend qu'il s'agit là de déterminer la quantité de certains gaz, par exemple du dioxyde de carbone, du méthane, etc., dégagés par une surface donnée, quantité qui est également appelée le flux de gaz dégagé par la surface. Cette quantité ou ce flux s'exprime en unité de quantité de gaz par unité de temps et de surface (par exemple en moles.minute⁻¹.m⁻², ou toute unité équivalente).

Pour mesurer cette quantité, différentes méthodes sont utilisées. Très fréquemment, on utilise des dispositifs de mesure constitués par une chambre configurée pour être posée sur la surface considérée de manière à délimiter un volume fermé par cette surface, et par un ou plusieurs analyseurs de gaz, reliés à la chambre, et permettant de mesurer la concentration des gaz qui se dégagent de la surface. A partir des mesures de la concentration et au moyen d'un modèle de calcul spécifique, la quantité de gaz rejetés par la surface peut être déterminée.

On connaît notamment les deux types de dispositifs suivants :
Dans les dispositifs dits 'à balayage', un gaz vecteur venant de l'extérieur est injecté dans la chambre à un débit régulier pendant la période de mesures ; ce gaz passe à travers la chambre et se charge ainsi des gaz émis par la surface de sol étudiée, le mélange de gaz ainsi obtenu est alors analysé à la sortie de la chambre par des moyens d'analyse de gaz adaptés. En utilisant un modèle de calcul propre à la méthode mise en oeuvre, les données recueillies permettent de déterminer la quantité de gaz rejetée par la surface de sol étudiée.

Dans les dispositifs dits 'à accumulation', aucun gaz vecteur venant de l'extérieur de la chambre n'est injecté dans celle-ci pendant la mesure. Dans ces dispositifs, on utilise un effet d'accumulation progressive de gaz émis par la surface à étudier dans le volume délimité par la chambre. La mesure est réalisée de la manière suivante :
on referme la chambre sur une surface dont on cherche à déterminer les émissions gazeuses ;
on prélève une partie du mélange gazeux contenu dans la chambre et on le transfère vers un analyseur de gaz adapté ;
on détermine l'évolution au cours du temps de la concentration de gaz à mesurer dans le volume délimité par la chambre.

Un exemple de dispositif à accumulation est présenté par le document EP0 807 822.

Dans un dispositif à accumulation, le mélange gazeux contenu dans la chambre a initialement une composition très proche de celle de l'atmosphère libre au-dessus de la surface à étudier : en général, de l'air. Ensuite, au fur et à mesure que la surface étudiée libère des gaz, ceux-ci s'accumulent dans la chambre ; la composition des gaz contenus dans la chambre change donc progressivement au fur et à mesure du temps.

La quantité de gaz libérés par la surface étudiée est donc déterminée en étudiant l'augmentation progressive de la concentration de certains gaz au cours du temps, à partir de l'instant de la fermeture de la chambre.

Dans ces dispositifs de mesure par accumulation, la mesure est donc réalisée en régime transitoire, à partir de la courbe d'augmentation de la concentration des gaz en fonction du temps. Par conséquent, chaque mesure du flux nécessite au préalable de renouveler en grande partie, et si possible complètement le mélange gazeux contenu dans la chambre.

Pour simplifier, dans la suite, l'opération de renouvellement du mélange gazeux contenu dans la chambre est appelée 'le renouvellement'.

Habituellement, pour réaliser un tel renouvellement, on soulève la chambre et on la maintient écartée de la surface étudiée pendant un temps suffisamment long pour que les gaz contenus dans la chambre se renouvellent complètement, et aient ainsi la même composition que l'atmosphère autour de la chambre.

Cette opération nécessite une intervention humaine et peut s'avérer à la longue fastidieuse, car certaines campagnes de mesure peuvent nécessiter de réaliser des séries de mesure en un point donné pendant plusieurs heures voire plusieurs jours. Il existe donc un besoin pour un dispositif de mesure permettant la réalisation de telles séries de mesures de manière automatisée, sans intervention humaine.

### OBJET ET RESUME DE L'INVENTION

Un premier objectif de l'invention est de proposer un dispositif fournissant des informations ou tout autre élément permettant de déterminer simplement les émissions gazeuses d'une surface de manière précise, sans nécessiter une intervention humaine pour chaque mesure.

Cet objectif est atteint grâce à un dispositif de mesure de concentrations d'au moins un gaz émis par une surface, comportant
une chambre configurée pour être posée sur ladite surface de manière à délimiter un volume fermé ;
un système d'analyse configuré pour mesurer des concentrations dudit au moins un gaz ;
un circuit d'analyse équipé d'une pompe, permettant de faire circuler un mélange gazeux contenu dans la chambre entre la chambre et le système d'analyse, en boucle fermée ;
au moins un passage d'entrée d'air et au moins un passage d'évacuation de gaz, agencés entre la chambre et une atmosphère environnante ;
un système de ventilation motorisé commandable ; et
une unité de commande électronique, configurée pour commander en alternance la réalisation :
d'étapes de renouvellement, durant lesquelles le système de ventilation injecte de l'air extérieur dans la chambre via ledit au moins un passage d'entrée d'air et extrait des gaz contenus dans celle-ci via ledit au moins un passage d'évacuation de gaz, de manière à renouveler le mélange gazeux contenu dans la chambre ;
d'étapes de mesure, pendant lesquelles le mélange gazeux contenu dans la chambre est mis en circulation dans le circuit d'analyse par la pompe, et dans le mélange gazeux ainsi mis en circulation, une concentration dudit au moins un gaz est mesurée au moins deux fois par le système d'analyse.

On comprend donc que le système de ventilation est raccordé audit au moins un passage d'entrée d'air, ainsi qu'audit au moins un passage d'évacuation de gaz.

De manière optionnelle, l'unité de commande électronique peut par ailleurs être configurée pour commander la réalisation d'étapes d'inactivité du dispositif, pendant lesquelles le dispositif reste au repos, si l'opérateur souhaite espacer dans le temps les étapes de mesure successives.

Le terme « air » est utilisé ici pour désigner le mélange de gaz qui constitue l'atmosphère environnant la chambre du dispositif de mesure (qui est en général, mais pas nécessairement, l'air de l'atmosphère terrestre). L'utilisation du terme « air » n'implique donc aucune valeur particulière quant à la composition du mélange de gaz considéré.

Grâce au circuit d'analyse qui l'alimente avec le mélange gazeux contenu dans la chambre, le système d'analyse permet de mesurer la concentration dans la chambre d'un ou plusieurs gaz, à savoir le ou les gaz que le système d'analyse de gaz permet de mesurer. Dans la suite, ces gaz sont dénommés « les gaz mesurés ».

Pour simplifier dans la description qui suit on pourra faire référence au pluriel à plusieurs gaz mesurés, mais cela devra être interprété comme visant également le cas où la concentration d'un seul gaz est mesurée.

Dans le dispositif de mesure selon l'invention, les passages d'entrée d'air et d'évacuation de gaz associés au système de ventilation motorisé permettent de renouveler partiellement ou complètement le mélange gazeux contenu dans la chambre. Ensuite, une fois l'étape de renouvellement réalisée, l'unité de commande électronique permet de réaliser une étape de mesure au cours de laquelle la concentration des gaz mesurés est mesurée de manière automatique au moins deux fois de suite par le système d'analyse.

Cette mesure est effectuée au moins deux fois de suite car, pour déterminer la valeur d'un flux de gaz dégagé par la surface étudiée, il est nécessaire de disposer d'au moins deux mesures de concentration dans la chambre à deux instants différents : la valeur de ce flux de gaz est calculée en fonction du changement de la concentration de ce gaz entre ces deux instants. La précision de ce calcul peut être augmentée en calculant la valeur du flux à partir de plus de deux mesures successives.

Les mesures de concentration de gaz successives sont réalisées de préférence peu de temps après que le mélange gazeux contenu dans la chambre a été renouvelé. Ainsi, ces mesures sont réalisées pendant une période pendant laquelle les concentrations de gaz mesurés augmentent rapidement dans la chambre, ce qui permet d'améliorer la précision du calcul du flux de gaz dégagé par la surface.

Dans le dispositif de mesure, l'unité de commande électronique permet de réaliser successivement et en alternance, de manière automatique, des étapes de renouvellement, des étapes de mesure, et éventuellement si l'opérateur le souhaite, des étapes (ou périodes) d'inactivité.

A partir des concentrations mesurées par le dispositif de mesure, il est possible d'obtenir une suite de valeurs des quantités (ou flux) de gaz dégagées par la surface étudiée en fonction du temps. Selon la manière dont le dispositif est utilisé, les différentes valeurs de flux ainsi obtenues peuvent être plus ou moins espacées dans le temps.

Lors de la mise en oeuvre du dispositif de mesure, le mélange gazeux contenu dans la chambre doit être renouvelé par ventilation forcée avant chaque étape de mesure (hormis éventuellement l'étape de mesure initiale, si celle-ci est réalisée juste après l'installation de la chambre). Malgré ces étapes de renouvellement, la mise en oeuvre du dispositif de mesure avantageusement ne nécessite aucun mouvement de la chambre. Cela lui confère plusieurs avantages, comparativement à d'autres solutions envisageables pour assurer le renouvellement du mélange gazeux contenu dans la chambre : la simplicité du point de vue mécanique, une faible sensibilité aux éléments extérieurs tels que le vent, la pluie, la chute de débris susceptibles de perturber son fonctionnement, une faible consommation de l'énergie, et un poids additionnel limité.

Le système de ventilation peut être réalisé de différentes manières.

Dans un mode de réalisation, le système de ventilation comporte au moins un ventilateur placé dans ledit au moins un passage d'entrée d'air et/ou ledit au moins un passage d'évacuation de gaz et configuré pour assurer un mouvement forcé de l'air dans ledit au moins un passage. De préférence, il comporte à la fois un ou plusieurs ventilateur(s) placé(s) dans le(s) passage(s) d'entrée d'air et servant à injecter de l'air dans la chambre, et un ou plusieurs ventilateur(s) placé(s) dans le(s) passage(s) d'évacuation de gaz et servant à évacuer le mélange gazeux contenu dans la chambre, car cette configuration est celle qui provoque la perturbation la plus faible quant au dégagement de gaz par la surface étudiée.

Dans un mode de réalisation, le système de ventilation comporte au moins une soupape placée dans ledit au moins un passage d'entrée d'air et/ou ledit au moins un passage d'évacuation de gaz et configurée pour permettre ou inversement pour empêcher une circulation de l'air dans ledit au moins un passage. Cette ou ces soupapes sont normalement ouvertes pendant les phases de renouvellement et fermées le reste du temps.

Dans un mode de réalisation, ledit au moins un passage d'entrée d'air et ledit au moins un passage d'évacuation de gaz sont agencés respectivement dans des parois opposées de la chambre. C'est cette configuration qui permet le renouvellement le plus complet du mélange gazeux contenu dans la chambre.

Dans un mode de réalisation, le dispositif de mesure comporte en outre un calculateur configuré pour déterminer un flux dudit au moins un gaz émis par la surface à partir des concentrations acquises lors des étapes de mesure. L'unité de commande électronique peut éventuellement constituer ce calculateur.

Par ailleurs, le premier objectif visé par la présente invention peut être atteint non seulement par le dispositif de mesure de concentrations de gaz émis par une surface défini précédemment, mais également par un dispositif de prélèvement d'échantillons pour mesurer des concentrations d'au moins un gaz émis par une surface, le dispositif comportant
une chambre configurée pour être posée sur ladite surface de manière à délimiter un volume fermé ;
un système de prélèvement d'échantillons configuré pour prélever des échantillons d'un mélange gazeux contenu dans la chambre ;
au moins un passage d'entrée de gaz et au moins un passage d'évacuation de gaz, agencés entre la chambre et l'atmosphère environnante ;
un système de ventilation motorisé commandable ; et
une unité de commande électronique configurée pour commander en alternance la réalisation :
d'étapes de renouvellement, durant lesquelles le système de ventilation injecte de l'air extérieur dans la chambre via ledit au moins un passage d'entrée d'air et extrait des gaz contenus dans celle-ci via ledit au moins un passage d'évacuation de gaz, de manière à renouveler le mélange gazeux contenu dans la chambre ;
d'étapes de prélèvement d'échantillons durant lesquelles le système de prélèvement d'échantillons prélève au moins deux échantillons du mélange gazeux contenu dans la chambre.

Comme précédemment, le système de ventilation est raccordé audit au moins un passage d'entrée d'air, ainsi qu'audit au moins un passage d'évacuation de gaz.

Ce dispositif de prélèvement d'échantillons présente donc les mêmes caractéristiques que le dispositif de mesure de concentrations présenté précédemment, sauf qu'au lieu de comporter un système d'analyse permettant de mesurer des concentrations de gaz et un circuit d'analyse pour acheminer le mélange gazeux jusqu'au système d'analyse, et au lieu que l'unité de commande électronique soit configurée pour faire réaliser des mesures de concentration comme indiqué précédemment, le dispositif de prélèvement d'échantillons comporte un système de prélèvement d'échantillons, et l'unité de commande électronique est configurée pour faire réaliser des étapes de prélèvement d'échantillons, en alternance avec les étapes de renouvellement. Ainsi, au lieu que la mesure des concentrations du ou des gaz mesurés soit faite en temps réel et in situ, elle est faite de manière différée, lorsque les échantillons prélevés lors des étapes de prélèvement d'échantillons sont portés à un laboratoire d'analyse pour mesurer la concentration des 'gaz mesurés'.

Bien qu'étant différée, la mesure des concentrations du ou des gaz mesurés dans les échantillons prélevés permet ensuite, comme avec le dispositif de mesure présenté précédemment, de calculer la valeur du ou de flux des gaz mesurés rejetés par la surface étudiée.

Par ailleurs, dans le dispositif de prélèvement d'échantillons, la chambre et le système de ventilation motorisé peuvent naturellement être également dotés des perfectionnements envisagés pour ceux-ci dans le dispositif de mesure de concentrations.

Un deuxième objectif de l'invention est de proposer un procédé fournissant des informations ou tout autre élément permettant de déterminer simplement les émissions gazeuses d'une surface de manière précise, sans nécessiter une intervention humaine sur site pour chaque mesure.

Cet objectif est atteint grâce à un procédé de mesure de concentrations d'au moins un gaz émis par une surface, suivant lequel
on dispose une chambre de telle sorte qu'elle délimite un volume fermé recouvrant ladite surface, et on procède en alternance à :
des étapes de renouvellement, durant lesquelles sans déplacer la chambre on injecte de l'air extérieur dans celle-ci et on extrait des gaz contenus dans celle-ci afin de renouveler un mélange gazeux contenu dans la chambre ; et
des étapes de mesure, durant lesquelles on fait circuler le mélange gazeux contenu dans ladite chambre dans un circuit d'analyse aménagé en boucle fermée entre la chambre et un système d'analyse, et dans le mélange ainsi mis en circulation, on mesure au moins deux fois une concentration dudit au moins un gaz.

Si l'opérateur souhaite espacer les mesures dans le temps, des étapes d'inactivité du dispositif peuvent être programmées.

Le procédé comporte donc, en alternance, une étape de renouvellement, une étape de mesure et, si l'opérateur le souhaite, une étape d'inactivité. L'étape de renouvellement est indispensable avant chaque étape de mesure ; elle est de préférence réalisée immédiatement avant l'étape de mesure.

Le procédé peut notamment être mis en oeuvre avec le dispositif de mesure décrit précédemment.

Avantageusement, ce procédé de mesure de concentrations de gaz fournit des mesures de concentrations des gaz mesurés qui permettent ensuite simplement de mesurer les flux de gaz émis par la surface. Aussi, par extension l'invention vise également un procédé de mesure de flux d'au moins un gaz émis par une surface, dans lequel on met en oeuvre le procédé de mesure de concentrations de gaz défini précédemment de manière à acquérir des concentrations dudit au moins un gaz émis par la surface, et on détermine un flux dudit au moins un gaz émis par la surface à partir des concentrations ainsi acquises.

Le deuxième objectif de l'invention peut en outre être également atteint par un procédé de prélèvement d'échantillons d'au moins un gaz émis par une surface, suivant lequel
on dispose une chambre de telle sorte qu'elle délimite un volume fermé recouvrant ladite surface, et on procède en alternance à des étapes de renouvellement, durant lesquelles sans déplacer la chambre on injecte de l'air extérieur dans celle-ci et on extrait des gaz contenus dans celle-ci afin de renouveler un mélange gazeux contenu dans la chambre ; et à des étapes de prélèvement d'échantillons, durant lesquelles on prélève au moins deux échantillons du mélange gazeux contenu dans la chambre.

Les concentrations dudit au moins un gaz émis par la surface peuvent être mesurées dans les échantillons prélevés, par exemple en laboratoire. A partir de ces mesures de concentrations, il est ensuite facile de calculer les flux de gaz dégagés par la surface.

Par suite, l'invention vise également un procédé de mesure de flux d'au moins un gaz émis par une surface, suivant lequel on met en oeuvre le procédé de prélèvement d'échantillons défini précédemment, de manière à prélever des échantillons du mélange gazeux contenu dans la chambre ; on mesure des concentrations dudit au moins un gaz émis par la surface dans chacun des échantillons prélevés ; et on détermine un flux dudit au moins un gaz émis par la surface à partir des concentrations ainsi mesurées.

### BREVE DESCRIPTION DES DESSINS

L'invention sera bien comprise et ses avantages apparaîtront mieux à la lecture de la description détaillée qui suit, de modes de réalisation représentés à titre d'exemples non limitatifs. La description se réfère aux dessins annexés, sur lesquels :
- la figure 1 est une vue schématique de dessus d'un dispositif de mesure de concentrations de gaz selon l'invention ;
- la figure 2 est une vue schématique de côté du dispositif de mesure de concentrations de gaz de la figure 1 ;
- la figure 3 est une vue schématique de côté d'un dispositif de prélèvement d'échantillons selon l'invention ;
- la figure 4 est un diagramme représentant les variations de concentration d'un gaz émis par une surface, mesurées par un dispositif de mesure de concentrations de gaz, pendant des étapes successives du procédé de mesure de concentrations selon l'invention ;
- la figure 5 est un diagramme représentant les étapes successives d'un procédé de mesure de concentrations de gaz selon l'invention ; et
- la figure 6 est un diagramme représentant les étapes successives d'un procédé de prélèvement d'échantillons selon l'invention.

### DESCRIPTION DETAILLEE DE L'INVENTION

### Structure d'un dispositif de mesure et d'un dispositif de prélèvement d'échantillons selon l'invention

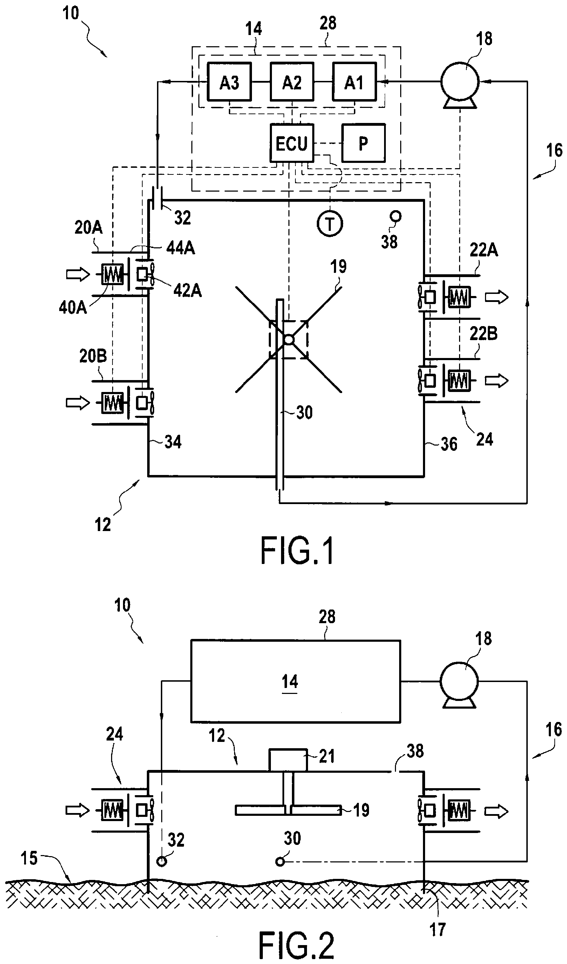

En référence aux figures 1 et 2, un exemple de dispositif de mesure de concentrations de gaz 10 selon l'invention va maintenant être présenté. Dans les différentes figures, les éléments (ou parties d'éléments) identiques ou similaires comportent la même référence numérique.

Le dispositif de mesure 10 comporte une chambre 12, un système d'analyse de gaz 14, un circuit 16 en boucle fermée équipé d'une pompe 18, deux passages d'entrée d'air 20A,20B, deux passages d'évacuation de gaz 22A,22B, un système de ventilation motorisé commandable 24, et une unité de commande électronique ECU. Le système d'analyse de gaz 14 comporte au moins un analyseur de gaz A1.

Dans le mode de réalisation présenté, la chambre 12 a la forme d'un parallélépipède rectangle. Cependant, elle peut prendre d'autres formes, par exemple un cylindre. La chambre est ouverte sur son côté inférieur. Comme on le voit sur la coupe de la figure 2, la chambre 12 est configurée pour être placée sur une surface 15 à examiner de manière à délimiter un volume fermé entre la surface 15 et la chambre 12. Dans cette position, le pourtour 17 de la chambre est installé de manière étanche sur la surface à examiner (par exemple, légèrement enfoncé dans le sol, comme c'est représenté sur la figure 2), pour permettre d'éviter tout échange de gaz entre la chambre 12 et l'atmosphère environnante.

Une fois placée sur la surface 15, la chambre 12 est prévue pour être sensiblement étanche, à l'exception du circuit 16 d'analyse de gaz et des passages d'entrée d'air 20A,20B et des passages d'évacuation 22A,22B, et d'un trou 38 situé dans une paroi de la chambre opposée à la surface 15.

Ce trou 38 de communication avec l'extérieur permet d'empêcher une surpression à l'intérieur de la chambre. Le diamètre du trou 38 est cependant choisi suffisamment petit pour éviter tout échange significatif de gaz entre le volume délimité par la chambre 15 et l'atmosphère environnante.

La chambre 12 contient également une hélice 19 qui assure le brassage du mélange gazeux contenu dans la chambre et est entraînée par un moteur 21 disposé sur le dessus de la chambre 12.

Le circuit 16 forme une boucle fermée. Ce circuit comprend une rampe d'aspiration 30 qui permet l'aspiration de gaz présents dans la chambre 12, des conduites de gaz qui permettent de conduire les gaz ainsi aspirés de la chambre 12 à la pompe 18, puis de la pompe 18 au système d'analyse de gaz 14, et enfin du système d'analyse de gaz 14 à une buse de réinjection 32. Cette buse 32 est aménagée dans la paroi de la chambre 12 et permet de réinjecter les gaz aspirés par la rampe 30 dans la chambre 12 après qu'ils ont transité à travers la pompe 18 et le système d'analyse de gaz 14.

Pendant les phases de mesure, la pompe 18 assure la mise en circulation des gaz contenus dans la chambre entre la chambre 12 et l'analyseur 14 via le circuit 16.

Le système d'analyse de gaz 14 doit permettre la mesure des concentrations de gaz circulant dans le circuit 16. Son fonctionnement peut être en grande partie similaire à celui décrit dans le document EP 0807822.

Dans le mode de réalisation présenté, le système d'analyse de gaz 14 comporte trois analyseurs de gaz référencés A1, A2 et A3, pour l'analyse par exemple respectivement de la concentration en méthane (CH₄), en dioxyde de carbone (CO₂) et en helium (He).

Le dispositif de mesure comporte également un capteur de pression P, un capteur de température T disposé dans la chambre 12, et une unité électronique ECU. Les analyseurs de gaz A1,A2,A3, le capteur P et l'unité de commande ECU sont placés dans un coffret de protection étanche 28.

D'autre part, pour assurer le renouvellement du mélange gazeux contenu dans la chambre 12 entre les mesures, le dispositif de mesure 10 comporte les deux passages d'entrée d'air 20A,20B, les deux passages d'évacuation de gaz 22A,22B, et le système de ventilation 24.

Pour simplifier, les passages d'entrée d'air et les passages d'évacuation de gaz sont appelés collectivement dans la suite 'les passages'. Chacun des passages 20A,20B et 22A,22B est agencé entre la chambre 12 et l'atmosphère environnant la chambre.

Ils sont installés sur des faces latérales de la chambre 12 (c'est-à-dire des faces verticales, autres que la face supérieure). Les passages d'entrée d'air 20A,20B sont situés du côté opposé aux passages d'évacuation de gaz 22A,22B, de manière à favoriser le renouvellement du mélange gazeux contenu dans la chambre. Dans le mode de réalisation présenté, les passages d'entrée d'air 20A et 20B sont placés côte à côte sur une première face latérale 34 de la chambre 12 ; les passages d'évacuation de gaz 22A et 22B sont placés côte à côte sur une deuxième face latérale 36 de la chambre 12. La face 36 est située du côté opposé à la face 34.

Dans le mode de réalisation présenté, le système de ventilation 24 comporte exactement les mêmes équipements dans chacun des passages ; par conséquent, la description de ces équipements va être faite simplement en référence à un passage, par exemple le passage d'entrée d'air 20A.

Ce passage 20A comporte une soupape commandable 40A et un ventilateur commandable 42A, tous deux reliés à l'unité ECU.

La soupape 40A et le ventilateur 42A sont placés dans un conduit de protection tubulaire 44A, fixé sur la paroi 34 de la chambre.

Comme indiqué précédemment, dans le mode de réalisation présenté, le dispositif de mesure 10 comporte quatre passages, et chacun d'eux est équipé d'une soupape, d'un ventilateur et d'un conduit de protection. Cependant, dans d'autres modes de réalisation le nombre de passages d'entrée d'air (et/ou d'évacuation de gaz) peut être réduit à un ou augmenté à strictement plus de deux. D'autre part, il n'est pas toujours nécessaire d'équiper à la fois les passages d'entrée d'air et les passages d'évacuation de gaz de ventilateurs : il peut être suffisant d'équiper soit le(s) passage(s) d'entrée d'air, soit le(s) passage(s) d'évacuation de gaz.

Enfin, l'unité de commande ECU assure différentes fonctions. Elle comporte tout d'abord un enregistreur qui enregistre les mesures de concentration de gaz effectuées par les analyseurs de gaz, ainsi que les mesures de pression et de température effectuées par les capteurs de pression P et de température T.

Elle constitue également un système de commande apte à gérer toutes les phases ou étapes de fonctionnement du dispositif. Pour permettre la gestion de ces phases de fonctionnement de manière automatique, l'unité ECU comporte un programmateur associé à des relais électriques qui permettent la commande des différents équipements commandés du dispositif, comme cela va être expliqué plus loin. Le programmateur permet notamment de programmer l'instant auquel chacune des opérations nécessaires pour la réalisation des étapes du procédé doit être réalisée (ouverture/fermeture des soupapes des passages d'entrée ou d'évacuation de gaz, mise en marche/arrêt de la pompe, des analyseurs, de l'hélice, etc.).

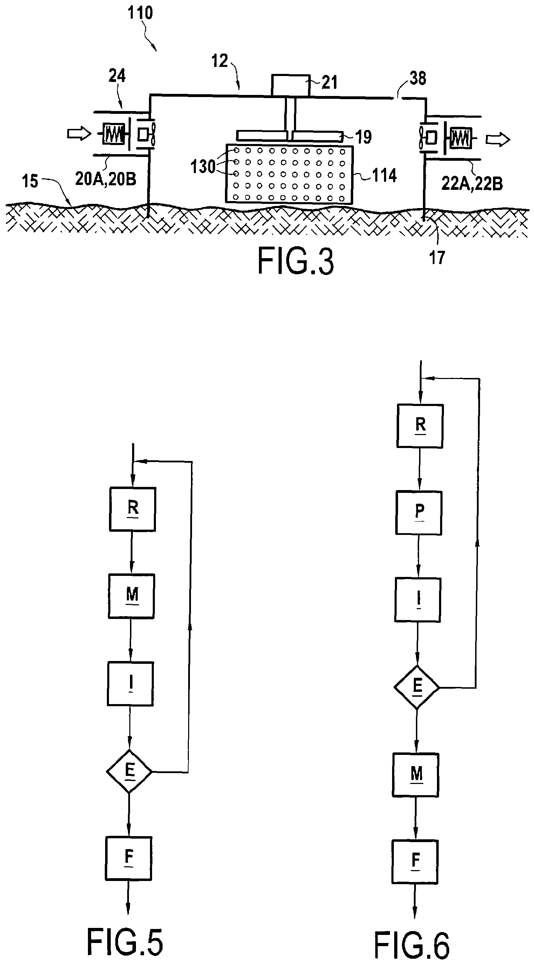

En référence à la figure 3, un exemple de dispositif de prélèvement d'échantillons 110 qui représente un autre aspect de l'invention va maintenant être présenté.

Le dispositif 110 de prélèvement d'échantillons est en grande partie identique au dispositif 10 de mesure de concentrations. Il comporte différents éléments identiques au dispositif 10, à savoir la chambre 12, les passages d'entrée d'air 20A,20B, les passages d'évacuation de gaz 22A,22B, et le système de ventilation motorisé commandable 24.

Cependant, dans le dispositif 110, le système d'analyse de gaz 14 et le circuit d'analyse 16 sont remplacés par un système de prélèvement d'échantillons 114. Sur la figure 3, le système 114 est représenté avec 50 orifices 130 de prélèvement d'échantillons.

De plus, l'unité de commande électronique ECU du dispositif 10 configurée pour faire réaliser des étapes de mesure de concentrations en alternance avec des étapes de renouvellement est remplacée dans le dispositif 110 par une unité de commande électronique (non représentée) configurée pour faire réaliser des étapes de prélèvement d'échantillons (par le système de prélèvement d'échantillons 114), en alternance avec des étapes de renouvellement.

### Procédé de mesure de concentrations de gaz ou de prélèvement d'échantillons, et de détermination des quantités de gaz dégagées par la surface étudiée

Le dispositif 10 permet de mettre en oeuvre du procédé de mesure de concentrations de gaz selon l'invention. Ce procédé peut être mis en oeuvre en répétant d'abord de manière itérative les étapes représentées schématiquement sur la figure 5 :
- étape R de renouvellement d'air pendant laquelle le mélange gazeux contenu dans la chambre 12 est renouvelé ;
- étape M de mesure des concentrations, pendant laquelle la concentration de chacun des gaz mesurés est mesurée, au moins deux fois ; et
- étape I d'inactivité, pendant laquelle aucune action n'est réalisée.

A l'issue de l'étape I, on évalue si l'ensemble des mesures prévues ont été réalisées ou non (étape E). Si ce n'est pas le cas, on reprend les étapes R, M, I.

Si c'est le cas, on peut alors déterminer le ou les flux de gaz mesurés dégagés par la surface 15 étudiée, aux différents instants où les étapes de mesure M ont été réalisées, à partir des valeurs de concentration mesurées précédemment (étape F).

Par ailleurs, le dispositif 110 permet quant à lui de mettre en oeuvre le procédé de prélèvement d'échantillons selon l'invention. Ce procédé peut être mis en oeuvre en répétant d'abord de manière itérative les étapes représentées schématiquement sur la figure 6 :
- étape R de renouvellement d'air pendant laquelle le mélange gazeux contenu dans la chambre 12 est renouvelé ;
- étape P de prélèvement d'échantillons, pendant laquelle on prélève au moins deux échantillons du mélange gazeux contenu dans la chambre ; et
- étape I d'inactivité, pendant laquelle aucune action n'est réalisée.

A l'issue de l'étape I, on évalue si l'ensemble des prélèvements d'échantillons prévus ont été réalisés ou non (étape E). Si ce n'est pas le cas, on reprend les étapes R, P, I.

Si c'est le cas, on peut alors réaliser les opérations suivantes :
- étape M de mesure des concentrations des gaz mesurés pour chacun des échantillons prélevés ; puis,
- étape F de calcul du ou des flux de gaz mesurés dégagés par la surface 15 étudiée, aux différents instants où les prélèvements d'échantillons ont été réalisés, à partir des valeurs de concentration mesurées à l'étape M.

Un exemple de mise en oeuvre du procédé de mesure de concentrations de gaz selon l'invention à l'aide du dispositif 10 présenté ci-dessus va maintenant être présenté plus en détail.

Pour commencer, on met en place la chambre 12 sur la surface à examiner 15 en veillant à ce que son pourtour 17 soit disposé de manière étanche à la surface 15 pour qu'il n'y ait pas d'échange de gaz sous les faces latérales de la chambre. La chambre 12 délimite alors un volume fermé dont la limite inférieure est la surface 15.

On programme par ailleurs l'unité de commande ECU de manière à réaliser de manière répétée (ou itérative) une étape R de renouvellement d'air, une étape M de mesure et une étape I de repos ou d'inactivité.

Au cours de chaque étape de renouvellement, sans déplacer la chambre 12, on injecte de l'air extérieur dans celle-ci et on extrait le mélange gazeux contenu dans celle-ci, ce qui permet de renouveler l'atmosphère contenu dans la chambre 12. L'hélice 19 est également activée pour faciliter le renouvellement.

Au cours de chaque étape de mesure, on fait circuler une partie du mélange gazeux contenu dans la chambre 12 via le circuit d'analyse de gaz 16. En circulant dans ce circuit, le gaz passe à travers le système d'analyse 14. Lors de ce passage dans le système d'analyse, les concentrations des gaz à mesurer sont déterminées par les analyseurs A1, A2 et A3. Ces concentrations sont mesurées au moins deux fois au cours de l'étape de mesure.

Cette série de mesures constitue une « étape de mesure » au sens de l'invention. Elle fournit les mesures de concentrations de gaz successives nécessaires pour déterminer le flux de gaz dégagé par la surface 15 pendant l'étape de mesure M considérée, et cela pour les différents gaz détectés par les analyseurs A1, A2 et A3.

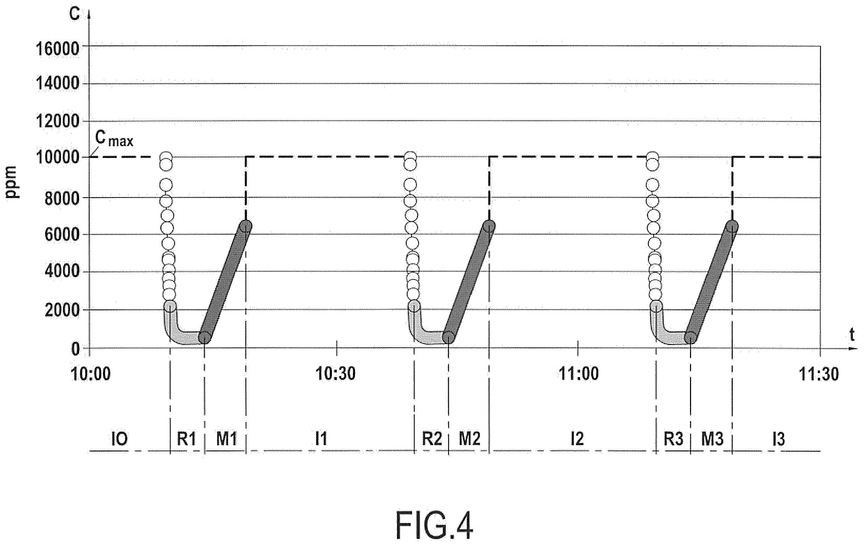

Un exemple d'enregistrements montrant une séquence d'étapes de renouvellement, de mesure et d'inactivité, réalisées de manière itérative, est illustré par la figure 4.

Cette figure représente, en fonction du temps t, les variations de la concentration C en dioxyde de carbone dans la chambre 12 qui résultent des rejets de dioxyde de carbone par la surface 15. La concentration C est exprimée en ppm (parties par million) ; elle est mesurée par un des analyseurs du système de mesure 14.

Comme on peut le voir sur cette figure, un certain temps avant 10h, on met en place la chambre 12 sur la surface 15. Par suite, l'air contenu dans la chambre se charge en gaz rejetés par la surface 15 et atteint une concentration en dioxyde de carbone supérieure au seuil haut de l'échelle de mesure de l'analyseur utilisé Cₘₐₓ, égale dans le cas présent à 10 000 ppm.

On réalise alors en alternance des étapes R de renouvellement du mélange de gaz contenus dans la chambre (étapes R1,R2,R3), et des étapes M de mesure (étapes M1,M2,M3). Dans le cas présent, une étape (optionnelle) d'inactivité I (étapes I1,I2,I3) est de plus programmée après chaque étape de mesure M.

On commence par une étape de renouvellement R1 pour assurer que la composition de l'atmosphère contenu dans la chambre, dès le début de la première étape de mesure M1, soit la plus proche possible de celle de l'air environnant la chambre 12.

Pour chaque étape de renouvellement R, on ouvre les soupapes des passages d'entrée d'air et d'évacuation de gaz et on actionne les ventilateurs contenus dans ces passages.

Pendant cette étape (comme pendant les étapes de mesure et d'inactivité), la chambre reste en place sur la surface 15. Le renouvellement de l'atmosphère contenu dans la chambre 12 est assuré par les quatre ventilateurs contenus dans les passages. Les deux ventilateurs des passages d'entrée d'air injectent de l'air frais extérieur dans la chambre 12, alors que les deux autres ventilateurs, situés dans les passages d'évacuation de gaz et situés du côté opposé de la chambre, aspirent les gaz contenus dans la chambre et les rejettent à l'extérieur de celle-ci.

Comme les gaz contenus dans la chambre sont ainsi évacués alors que de l'air frais est injecté, la concentration C en dioxyde de carbone (ou des autres gaz rejetés par la surface 15) dans la chambre 12 chute puis se stabilise à un niveau proche de celui de l'air environnant.

Après un temps suffisant pour que le renouvellement de l'atmosphère dans la chambre soit complet, les ventilateurs sont arrêtés et les soupapes fermées, et une étape de mesure M est réalisée.

Pendant cette étape de mesure (étapes M1,M2,M3), du fait des rejets de dioxyde de carbone par la surface étudiée 15, la concentration en dioxyde de carbone dans la chambre 12 augmente progressivement.

Pendant cette période, on réalise au moins deux mesures. Pour cela, on fait circuler le mélange de gaz contenu dans la chambre 12 dans le circuit 16 grâce à la pompe 18 ; le mélange de gaz traverse le système d'analyse 14 et donc les analyseurs A1, A2 et A3 ; ceux-ci déterminent la concentration des gaz étudiés dans ce mélange de gaz, et transmettent les valeurs mesurées à l'unité ECU. L'unité ECU dispose ainsi des valeurs de concentration des gaz mesurés en fonction du temps. Ces informations lui permettent ensuite de calculer le flux de gaz émis par la surface 15, de manière connue en soi.

Pour chacun des gaz mesurés, chaque flux surfacique F de gaz émis est calculé en prenant en compte la variation dans le temps de la concentration du gaz considéré dans le volume V de la chambre 12 par rapport à l'aire S de la surface couverte 15.

Un facteur de correction exprimant l'influence du dispositif de mesure sur le flux peut être pris en compte dans ce calcul. Ce facteur dépend principalement des caractéristiques techniques du dispositif de mesure utilisé et de l'environnement dans lequel sont réalisées les mesures (par exemple, la nature du sol étudié et son humidité). Le facteur de correction est déterminé préalablement lors des essais de validation métrologique du dispositif en laboratoire.

Si nécessaire, on prend également en compte les conditions de température et de pression ambiante sur le lieu de mesure, qui sont mesurées par les capteurs de température T et de pression P.

A la fin d'une étape de mesure, les opérations de mesure de concentration successives sont interrompues en arrêtant la pompe 18 et l'enregistreur de l'unité ECU, lorsqu'un nombre suffisant de mesures de concentration des gaz rejetés a été enregistré. La durée de l'étape de mesure est ajustée et programmée en fonction de la nature des gaz à mesurer, de l'intensité de l'émission et des caractéristiques des analyseurs.

Le dispositif de mesure reste alors à l'arrêt pendant une période d'inactivité (I1,I2,I3).

Ensuite, à l'instant souhaité pour la reprise des mesures, l'unité ECU lance une nouvelle étape R de renouvellement du mélange gazeux contenu dans la chambre, suivie par une nouvelle étape M de mesure.

Les étapes de renouvellement, de mesure et les étapes optionnelles d'inactivité sont réalisées de manière itérative le nombre de fois voulu. Seuls trois cycles de mesure (chaque cycle comprenant une étape de renouvellement R1,R2,R3, une étape de mesure M1,M2,M3 et une étape d'inactivité I1,I2,I3) sont représentés sur la figure 4.

La durée habituelle d'un cycle complet de renouvellement et de mesure est d'environ 10 minutes. Elle est définie par la durée de l'étape de renouvellement (environ 5 minutes), ainsi que par la sensibilité des analyseurs et par l'intensité de l'émission des gaz par la surface étudiée, qui déterminent la durée de l'étape de mesure (généralement, environ 5 minutes). Les cycles de mesure sont programmés de manière à être réalisés successivement, avec ou sans période d'inactivité entre deux cycles. Les périodes d'inactivité peuvent être aussi longues que nécessaire, par exemple une heure, une semaine, un mois...

Toutes les étapes de fonctionnement du dispositif 10 décrites précédemment peuvent être programmées à l'avance de telle sorte que l'unité ECU les réalise suivant un programme prédéterminé. La gestion de toutes ces étapes de fonctionnement est ainsi entièrement automatisée.

Par ailleurs, si les analyseurs de gaz le permettent (possibilité de démarrage et de calibrage sans intervention de l'opérateur, durée limitée de chauffe ou de stabilisation etc.), ceux-ci peuvent être mis en veille voire arrêtés entre les étapes de mesure, pour économiser de l'énergie. Dans le cas contraire, ils fonctionnent en continu. De plus, de manière similaire, le capteur de température dans la chambre et celui de pression peuvent aussi fonctionner en continu, pour mieux apprécier les conditions extérieures durant l'étape de mesure. Ceci implique aussi généralement le fonctionnement en continu de l'unité de commande ECU.

Quoique la présente invention ait été décrite en se référant à un exemple de réalisation spécifique, il est évident que différentes modifications et changements peuvent être effectués sans sortir de la portée générale de l'invention telle que définie par les revendications. Par conséquent, la description et les dessins doivent être considérés dans un sens illustratif plutôt que restrictif.

## Revendications

1. Dispositif (10) de mesure de concentrations d'au moins un gaz émis par une surface (15), comportant
une chambre (12) configurée pour être posée sur ladite surface de manière à délimiter un volume fermé ;
un système d'analyse (14) configuré pour mesurer des concentrations dudit au moins un gaz ;
un circuit d'analyse (16) équipé d'une pompe (18), permettant de faire circuler un mélange gazeux contenu dans la chambre entre la chambre et le système d'analyse, en boucle fermée ;
le dispositif **se caractérisant en ce qu'**il comporte en outre :
au moins un passage d'entrée de gaz (20A,20B) et au moins un passage d'évacuation de gaz (22A,22B), agencés entre la chambre (12) et une atmosphère environnante ;
un système de ventilation motorisé commandable (24) ; et
une unité de commande électronique (ECU), configurée pour commander en alternance la réalisation :
d'étapes de renouvellement, durant lesquelles le système de ventilation (24) injecte de l'air extérieur dans la chambre (12) via ledit au moins un passage d'entrée d'air (20A,20B) et extrait des gaz contenus dans celle-ci via ledit au moins un passage d'évacuation de gaz (22A,22B), de manière à renouveler le mélange gazeux contenu dans la chambre (12) ; et
d'étapes de mesure, pendant lesquelles le mélange gazeux contenu dans la chambre est mis en circulation dans le circuit d'analyse (16) par la pompe (18), et dans le mélange gazeux ainsi mis en circulation, une concentration dudit au moins un gaz est mesurée au moins deux fois par le système d'analyse (14).

2. Dispositif (110) de prélèvement d'échantillons pour mesurer des concentrations d'au moins un gaz émis par une surface (15), le dispositif comportant
une chambre (12) configurée pour être posée sur ladite surface de manière à délimiter un volume fermé ;
un système de prélèvement d'échantillons (114) configuré pour prélever des échantillons d'un mélange gazeux contenu dans la chambre ;
le dispositif **se caractérisant en ce qu'**il comporte en outre :
au moins un passage d'entrée de gaz (20A,20B) et au moins un passage d'évacuation de gaz (22A,22B), agencés entre la chambre (12) et une atmosphère environnante ;
un système de ventilation motorisé commandable (24) ; et
une unité de commande électronique (ECU), configurée pour commander en alternance la réalisation :
d'étapes de renouvellement, durant lesquelles le système de ventilation (24) injecte de l'air extérieur dans la chambre (12) via ledit au moins un passage d'entrée d'air (20A,20B) et extrait des gaz contenus dans celle-ci via ledit au moins un passage d'évacuation de gaz (22A,22B), de manière à renouveler le mélange gazeux contenu dans la chambre (12) ; et
d'étapes de prélèvement d'échantillons durant lesquelles le système de prélèvement d'échantillons (114) prélève au moins deux échantillons du mélange gazeux contenu dans la chambre.

3. Dispositif selon la revendication 1 ou 2, dont le système de ventilation (24) comporte au moins un ventilateur (42A) placé dans ledit au moins un passage d'entrée d'air (20A) et/ou ledit au moins un passage d'évacuation de gaz et configuré pour assurer un mouvement forcé de l'air dans ledit au moins un passage.

4. Dispositif selon l'une quelconque des revendications 1 à 3, dont le système de ventilation (24) comporte au moins une soupape (40A) placée dans ledit au moins un passage d'entrée d'air (20A) et/ou ledit au moins un passage d'évacuation de gaz et configurée pour permettre ou inversement pour empêcher une circulation de l'air dans ledit au moins un passage.

5. Dispositif selon l'une quelconque des revendications 1 à 4, dans lequel ledit au moins un passage d'entrée d'air et ledit au moins un passage d'évacuation de gaz sont agencés respectivement dans des parois opposées (34,36) de la chambre.

6. Procédé de mesure de concentrations d'au moins un gaz émis par une surface (15), suivant lequel
on dispose une chambre (12) de telle sorte qu'elle délimite un volume fermé recouvrant ladite surface, et
on procède en alternance à :
des étapes de renouvellement, durant lesquelles sans déplacer la chambre on injecte de l'air extérieur dans celle-ci et on extrait des gaz contenus dans celle-ci afin de renouveler un mélange gazeux contenu dans la chambre ; et
des étapes de mesure, durant lesquelles on fait circuler le mélange gazeux contenu dans ladite chambre dans un circuit d'analyse (16) aménagé en boucle fermée entre la chambre et un système d'analyse (14), et dans le mélange gazeux ainsi mis en circulation, on mesure au moins deux fois une concentration dudit au moins un gaz.

7. Procédé de mesure de flux d'au moins un gaz émis par une surface (15), dans lequel on met en oeuvre le procédé de mesure selon la revendication 6 de manière à acquérir des concentrations dudit au moins un gaz émis par la surface, et on détermine un flux dudit au moins un gaz émis par la surface à partir des concentrations ainsi acquises.

8. Procédé de prélèvement d'échantillons d'au moins un gaz émis par une surface (15), suivant lequel
on dispose une chambre (12) de telle sorte qu'elle délimite un volume fermé recouvrant ladite surface, et
on procède en alternance à :
des étapes de renouvellement, durant lesquelles sans déplacer la chambre on injecte de l'air extérieur dans celle-ci et on extrait des gaz contenus dans celle-ci afin de renouveler un mélange gazeux contenu dans la chambre ; et
des étapes de prélèvement d'échantillons durant lesquelles on prélève au moins deux échantillons du mélange gazeux contenu dans la chambre.

9. Procédé de mesure de flux d'au moins un gaz émis par une surface (15), dans lequel on met en oeuvre le procédé de prélèvement d'échantillons selon la revendication 8 de manière à prélever des échantillons du mélange gazeux contenu dans la chambre ; on mesure des concentrations dudit au moins un gaz émis par la surface dans chacun des échantillons prélevés ; et on détermine un flux dudit au moins un gaz émis par la surface à partir des concentrations ainsi mesurées.
